# EUROPÄISCHE PATENTSCHRIFT

(11) **EP 0 492 326 B1**
(45) Veröffentlichungstag und Bekanntmachung des Hinweises auf die Patenterteilung: **01.03.1995**
(21) Anmeldenummer: 91121465.8
(22) Anmeldetag: 14.12.1991
(51) Int. Cl.: G01N 35/00, G01N 33/52

(54) **Testträger-Analysesystem**
Test strip analysing system
Analyseur pour bandes de test

(30) Priorität: 27.12.1990 DE 4041905
(43) Veröffentlichungstag der Anmeldung: 01.07.1992
(73) Patentinhaber: BOEHRINGER MANNHEIM GMBH, 68298 Mannheim (DE)
(72) Erfinder: Markart, Ernst, Dipl.-Ing., W-8000 München 60 (DE); Bolduan, Franz Dr.Dipl. Phys., D-6800 Mannheim 1 (DE); Schreiber, Jörg, Dr. Dipl. Phys., D-6805 Heddesheim (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 317 325
- EP-A- 0 353 589
- EP-A- 0 376 109
- EP-A- 0 383 322
- EP-A- 0 405 091
- GB-A- 2 096 314
- US-A- 4 476 149

## Beschreibung

Die Erfindung betrifft ein Testträger-Analysesystem zur Analyse eines Bestandteils einer flüssigen Probe, insbesondere einer Körperflüssigkeit. Zu dem System gehören drei Bestandteile, nämlich Testträger, die in einer oder mehreren Testschichten Reagenzien enthalten, deren Reaktion mit der Probe zu einer physikalisch nachweisbaren Veränderung in einer Nachweisschicht führt, Codeträger, die in maschinenlesbarer Form einen Auswertecode aufweisen, der für die Auswertung der physikalisch nachweisbaren Veränderung benötigt wird und ein Auswertegerät, mit dem die physikalisch nachweisbare Veränderung gemessen und in das Analyseergebnis umgerechnet wird.

Testträger-Analysesysteme sind insbesondere in der Medizin für die Analyse von Urin und Blut gebräuchlich. Die Testträger haben meist die Form von Teststreifen. Aber auch andere Formen von Testträgern werden verwendet, beispielsweise flache, näherungsweise quadratische Plättchen, in deren Mitte ein Testfeld angeordnet ist.

Die Testschichten werden mit der Probe in Kontakt gebracht, was bei der Untersuchung von Urin im allgemeinen durch Eintauchen geschieht. Bei der Untersuchung von Blut wird üblicherweise ein Blutstropfen aufgetropft.

Die physikalisch nachweisbare Veränderung ist meist ein Farbumschlag oder eine andere optisch nachweisbare Eigenschaft, beispielsweise eine Fluoreszenz. Es sind jedoch auch Testträger bekannt, die auf anderen, beispielsweise elektrochemischen Prinzipien basieren, wobei die physikalisch nachweisbare Veränderung sich auf einen elektrischen Strom oder eine elektrische Spannung bezieht.

Das Auswertegerät verfügt über eine Testträgeraufnahme zur Positionierung eines auszuwertenden Testträgers in einer Meßposition und eine Meßvorrichtung zur Messung der physikalisch nachweisbaren Veränderung. Im Falle eines Farbumschlages enthält die Meßvorrichtung ein Reflexionsphotometer zur Bestimmung des diffusen Reflexionsvermögens (Reflektivität) der Nachweisschicht. Im Falle elektrochemischer Testträger enthält die Meßvorrichtung eine entsprechende Strom- oder Spannungsmeßschaltung. In jedem Fall bestimmt sie ein Meßsignal R, aus dem sich die gesuchte Konzentration C ermitteln läßt.

Die Testträger sind üblicherweise spezifisch für eine bestimmte Analyse geeignet, d.h. ein Testträgertyp dient zur Feststellung der Konzentration eines bestimmten Bestandteiles einer Körperflüssigkeit, der als Parameter bezeichnet wird. Für einen bestimmten Testträgertyp, beispielsweise zur Analyse von Glucose oder Cholesterin in Blut, besteht ein bestimmter Zusammenhang zwischen dem Meßsignal R und der Konzentration C. Dieser wird als Auswertekurve bezeichnet.

Hierbei besteht allerdings bereits seit den Anfängen der quantitativen Analytik mit Hilfe von Testträgern ein besonderes Problem. Die Testträger werden nämlich chargenweise produziert und es ist in aller Regel nicht möglich, den Herstellungsvorgang so exakt reproduzierbar zu gestalten, daß bei hohen Anforderungen an die Genauigkeit der Analyse die gleiche Auswertekurve für verschiedene Herstellungschargen verwendet werden kann. Zur Lösung dieses Problems sind bereits eine Vielzahl von Vorschlägen gemacht worden.

Ein gebräuchlicher Weg ist die Eichung mit Hilfe von Kalibrationsflüssigkeiten bekannter Konzentration. Üblicherweise ist bei solchen System in dem Auswertegerät eine mittlere Auswertekurve abgespeichert, welche von Fall zu Fall aufgrund von Kalibrationsmessungen korrigiert werden kann. Dieses Verfahren ist jedoch zeitraubend und kompliziert und deswegen insbesondere bei Analysesystemen, die zur Anwendung durch Laien vorgesehen sind (sogenanntes "Home-monitoring"), auf die sich die Erfindung in besonderem Maße bezieht, schlecht geeignet.

Um hier Abhilfe zu schaffen wurden Analysesysteme entwickelt, zu denen neben den Testträgern und dem Auswertegerät spezielle Codeträger gehören, die die für die Auswertung erforderlichen Auswerteinformationen enthalten. Bei einem der ersten Systeme zur Analyse von Glucose, welches noch analogelektronisch arbeitete, wurden als Codeträger in diesem Sinne auswechselbare Skalenscheiben verwendet, die den jeweiligen Teststreifenpackungen beigegeben waren und deren Graduierung auf die jeweilige chargenspezifische Auswertekurve abgestimmt war. Später wurde der Auswertecode in maschinenlesbarer Form codiert. Bei manchen Geräten wurde ein read-only-memory (ROM)-Halbleiterbaustein verwendet, der allerdings keine chargenspezifische, sondern nur eine parameterspezifische Auswertekurve enthielt. Bei einem besonders weit verbreiteten System besteht der Codeträger aus einem Film mit einem Barcode, der eine chargenspezifische Auswertekurve enthält. Das Auswertegerät weist in solchen Fällen eine Codelesevorrichtung zum Lesen des Auswertecodes und einen Speicher zur Speicherung der in dem Auswertecode codierten Auswerteinformation auf. Das Meßsignal wird unter Berücksichtigung dieser Information in das Analyseergebnis umgewandelt.

Die Codeträger werden bei derartigen Systemen üblicherweise den Testträgerpackungen beigefügt. Wenn eine neue Packung Testträger angebrochen wird, muß der Benutzer jeweils den Codeträger in das Auswertegerät einlegen, damit die Auswerteinformation gelesen und abgespeichert werden kann. Diese Information kann dann beliebig oft für die Testträger der gleichen Packung verwendet werden. Dabei besteht aber keine Sicherheit, daß nicht versehentlich ein Testträger verwendet wird, der nicht zu der Charge gehört, deren Auswertekurve gerade abgespeichert ist.

Dieses Problem kann durch eine positive Chargenidentifikation gelöst werden, bei der sowohl das für die Analyse verwendete Element als auch der Codeträger einen Chargenidentifikationscode aufweisen, der jeweils von dem Auswertegerät gelesen und verglichen wird. Dies ist beispielsweise für ein System zum Testen von Allergien aus der EP-A-0 353 589 bekannt. Die dort beschriebene Ausführungsform eignet sich jedoch nur für verhältnismäßig große Laborsysteme.

Kein Verwechslungsrisiko besteht auch bei Analysesystemen, bei denen der Auswertecode auf den Testträgern selbst angebracht ist und vor jeder Auswertung gelesen wird. In der EP-A-73 056 bzw. dem US-Patent 4 592 893 ist ein Teststreifen beschrieben, der auf seiner Unterseite einen Barcode mit einer chargenspezifischen Auswertekurve enthält. Die EP-A-132 790 (entsprechend US-A-4 578 716) befaßt sich mit der Speicherung der Information in einer auf den Testträgern befindlichen Magnetschicht. Diese Systeme sind gegen Fehlbedienungen praktisch vollständig gesichert. Dieser Vorteil wird jedoch mit einem hohen Aufwand bei der Herstellung der Testträger erkauft, weil auf jede Charge ein spezifischer Code verhältnismäßig hoher Informationsdichte unter den für die Teststreifenherstellung typischen schwierigen Randbedingungen aufgebracht werden muß. Auf diese Probleme wird in den genannten Schriften im einzelnen eingegangen.

Der Erfindung liegt die Aufgabe zugrunde, ein Testträger-Analysesystem zur Verfügung zu stellen, welches eine genaue Analyse unter Berücksichtigung der chargenspezifischen Auswertekurve ohne Eichung durch den Benutzer ermöglicht und bei dem ohne wesentlich erhöhte Herstellkosten sowohl hinsichtlich der Testträger als auch hinsichtlich des Gerätes Fehlbedienungen praktisch ausgeschlossen sind.

Die Aufgabe wird bei einem mit separaten Testträgern und Codeträgern arbeitenden Analysesystem durch die Kombination folgender Maßnahmen gelöst. Der Auswertecode auf den Codeträgern ist ein zweispuriger Code mit getrennter Taktspur und Datenspur. Auf den Testträgern ist ein chargenspezifischer Identifikationscode in Form eines Barcode vorgesehen, dessen Codestriche über die gesamte Breite der Testträger verlaufen. Die Codelesevorrichtung ist an der Testträgeraufnahme so angeordnet, daß sowohl ein Testträger als auch ein Codeträger wahlweise eingeschoben und deren Codes während des Einschiebens und/oder Herausziehens gelesen werden können. Die Codeleseeinrichtung weist zwei sowohl seitlich als auch in der Einschubrichtung gegeneinander versetzte Leseeinheiten auf. Das Auswertegerät ist mit einer Vergleichseinheit ausgestattet, die zum Vergleich des Identifikationscodes der Testträger und der Chargenidentifikation der Auswertekurve und damit zur Überprüfung der Zuordnung der Charge des jeweils auszuwertenden Testträgers und der abgespeicherten Auswerteinformation dient.

Die Erfindung ermöglicht eine positive Identifikation des Auswertecodes und demzufolge eine praktisch vollständige Sicherung gegen Auswertefehler, die durch Verwendung einer falschen Auswertekurve entstehen könnten. Dies wird praktisch ohne zusätzlichen Aufwand erreicht. Es kann eine einzige Codelesevorrichtung zum Lesen der Codes sowohl auf den Testträgern als auch auf den Codeträgern verwendet werden. Beide Codes sind selbsttaktend und ermöglichen deswegen auch bei manueller Bewegung der Code- bzw. Testträger eine hohe Lesesicherheit unabhängig von der Geschwindigkeit der Bewegung des Codes.

Die beiden seitlich gegeneinander versetzten Leseeinheiten sind so angeordnet, daß eine auf die Taktspur und die andere auf die Datenspur des Auswertecodes gerichtet ist. Der zweispurige Code ist besonders vorteilhaft im Zusammenhang mit Testträger-Analysesystemen, weil er eine sehr hohe Informationsdichte ermöglicht und dadurch sehr kleine Codeträger verwendet werden können. Dies wiederum ist erforderlich, weil zunehmend kleinere Testträger verwendet werden, und bei Verwendung eines einzigen Einschubweges die nutzbare Länge der Codeträger von der entsprechenden Länge der Testträger abhängig ist.

Die Tatsache, daß der Code auf den Testträgern nur die Chargenidentifikation enthalten muß und als Barcode mit durchgängigen Codestrichen ausgebildet ist, ermöglicht es, die Aufbringung des Codes ohne wesentlichen Mehraufwand in den üblichen Herstellungsprozeß von Teststreifen zu integrieren. Dadurch, daß die Leseeinheiten auch in Richtung des Einschubweges (also in Leserichtung des Codes) gegeneinander versetzt sind, kann der Identifikationscode auf den Teststreifen selbsttaktend gelesen werden, ohne daß eine gesonderte Taktspur erforderlich ist oder hinsichtlich des Gerätes aufwendige Maßnahmen ergriffen werden müssen. Dies wird weiter unten näher erläutert.

Der Barcode läßt sich verhältnismäßig einfach auf die Codeträger und die Testträger aufbringen. Außerdem sind Barcode-Lesevorrichtungen einfach und kostengünstig. Dies ist besonders wichtig, weil die Erfindung insbesondere für kleine, leichte, tragbare Handgeräte geeignet ist, wie sie insbesondere von Diabetikern verwendet werden.

Zum Lesen des Codes ist eine geführte Relativbewegung zwischen Testträger und Codelesevorrichtung vorteilhaft. Sie läßt sich zweckmäßigerweise dadurch realisieren, daß die Testträgeraufnahme eine Führung aufweist, die so gestaltet ist, daß ein Testträger entlang einem Einschubweg in Richtung auf die Meßposition manuell eingeschoben werden kann, an dem die Codelesevorrichtung angeordnet ist.

Zweckmäßigerweise sind die Abmessungen der Codeträger und der Testträger so aufeinander abgestimmt, daß sie gemeinsam verpackt werden können. Zum Beispiel werden Teststreifen üblicherweise in Röhren geliefert und es ist praktisch, wenn diese auch einen streifenförmigen Codeträger enthalten. Dies gilt insbesondere bei Analysesystemen, die mit verschiedenen Testträgertypen zur Bestimmung verschiedener Parameter arbeiten und bei denen deshalb jeweils die passenden Codeträger für den Parameter vor der Auswertung gelesen werden müssen. Bei solchen Mehrparameter-Systemen wäre es sehr unhandlich, wenn die Codeträger getrennt von den Testträgern verpackt wären.

Bei Mehrparameter-Systemen enthält der Identifikationscode auf den Testträgern und der Auswertecode auf den Codeträgern jeweils Informationen über den Testträgertyp (d.h. den Parameter), so daß nicht nur die richtige Zuordnung der Charge, sondern auch die richtige Zuordnung des Testträgertyps zu der jeweiligen Auswertekurve geprüft bzw. die jeweils benötigte Auswertekurve aus dem Speicher aufgerufen werden kann.

Die Erfindung wird im folgenden anhand eines in den Figuren schematisch dargestellten Ausführungsbeispiels näher erläutert; es zeigen:
- Fig. 1: ein erfindungsgemäßes Analysesystem in perspektivischer Darstellung
- Fig. 2: eine Aufsicht auf die Codierungsseite (Unterseite) eines Testträgers,
- Fig. 3: eine Aufsicht auf die Codierungsseite eines Codeträgers,
- Fig. 4: einen Schnitt durch den Testträgeraufnahmebereich eines Auswertegerätes in Einschubrichtung,
- Fig. 5: einen Schnitt entlang der Linie V-V in Fig. 4 (senkrecht zur Einschubrichtung),
- Fig. 6: eine Prinzipskizze zur Erläuterung der Arbeitsweise einer Codelesevorrichtung.

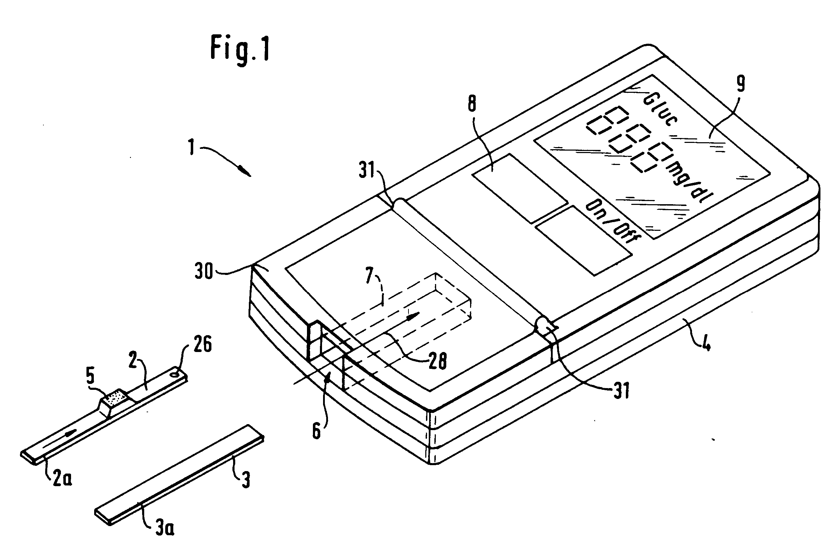

Figur 1 zeigt ein Testträger-Analysesystem 1, bestehend aus Testträgern 2, Codeträgern 3 und einem Auswertegerät 4.

Der Testträger 2 und der Codeträger 3 sind streifenförmig ausgebildet. Sie haben gleiche Abmessungen. Ihre Tragschicht 2a bzw. 3a wird jeweils von einer steifen Kunststoffolie gebildet. Der Testträger 2 weist im dargestellten Beispielsfall nur ein einziges Testfeld 5 auf, welches aus mehreren Testschichten bestehen kann.

Das Auswertegerät 4 hat eine Einführöffnung 6, durch die wahlweise ein Testträger 2 oder ein Codeträger 3 in die im Inneren des Gerätes befindliche, in der Figur gestrichelt angedeutete Testträgeraufnahme 7 eingeschoben werden kann. Zur Bedienung des Gerätes ist ein Tastenfeld 8 vorgesehen. Die Meßergebnisse werden in einem Display 9 angezeigt.

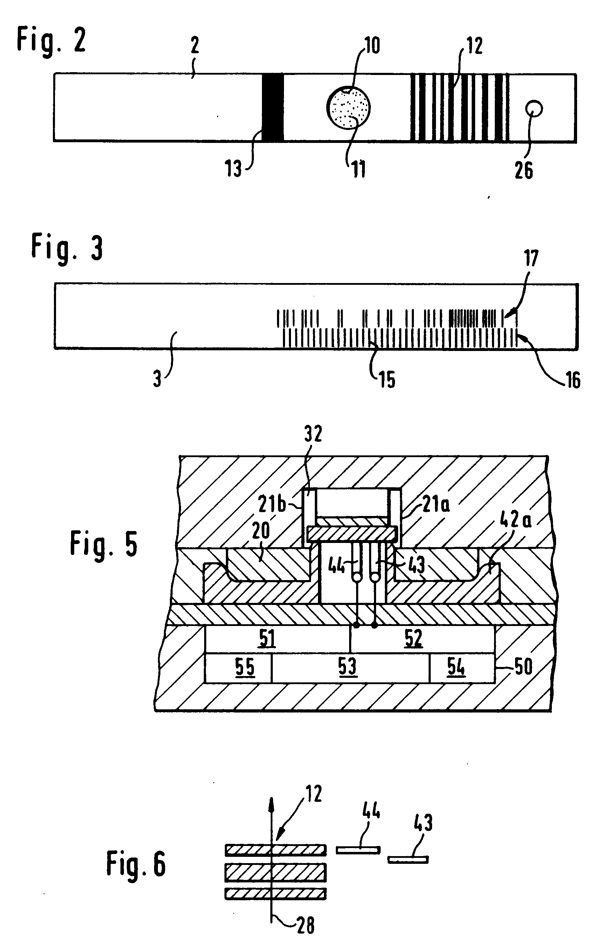

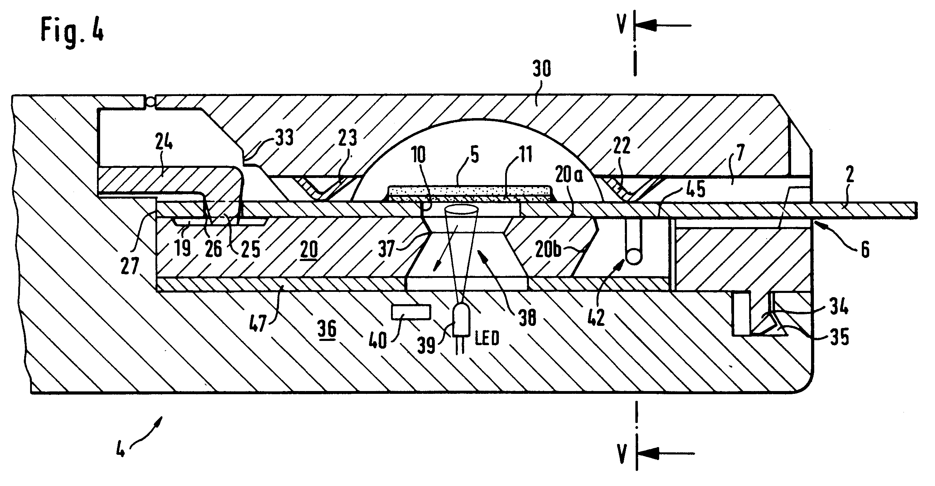

Wie in Figur 2 zu erkennen ist, hat der Testträger 2 auf seiner Unterseite eine kreisförmige Ausnehmung 10, durch die die unterste Schicht 11 des Testfeldes 5 zu erkennen ist. Hierbei handelt es sich um die Nachweisschicht, auf der infolge der Reaktion der in dem Testfeld 5 enthaltenen Reagenzien mit einem auf seine Oberseite aufgegebenen Probentropfen ein für die Analyse charakteristischer Farbumschlag stattfindet. Dieser Farbumschlag wird von dem Auswertegerät 4 gemessen und in das Analyseergebnis umgerechnet.

Auf der Unterseite des Testträgers 2 erkennt man weiterhin einen Identifikationscode 12 und eine balkenförmige Markierung 13, deren Streifen sich jeweils quer über den gesamten Testträger erstrecken.

Wie in Figur 3 zu erkennen ist, weist der Codeträger 3 auf seiner Codierungsseite (welche beim Einführen in das Auswertegerät 4 die Unterseite ist) einen Auswertecode 15 auf, der aus zwei parallel zueinander verlaufenden Spuren, nämlich eine Taktspur 16 und einer Datenspur 17 besteht.

In Figur 4 und 5 ist der vordere Teil des Auswertegerätes 4 in zwei zueinander senkrechten stark schematisierten Schnittzeichnungen dargestellt. Die Testträgeraufnahme 7 umfaßt eine Testträgerauflage 20, seitliche Führungen 21a, 21b und zwei Andruckfedern 22,23. Durch diese Elemente wird sowohl ein Testträger 2 als auch ein Codeträger 3, der manuell in die Einführöffnung 6 eingeführt wird, entlang einem Einschubweg 28 (Fig. 1) geführt. Wenn ein Testträger sich in der in Figur 4 dargestellten Meßposition befindet, greift ein in Richtung auf die Testträgerauflage 20 vorgespanntes Rastelement 24 mit einem Rastvorsprung 25 in eine passende Ausnehmung 26 des Testträgers 2 ein. Die Ausnehmung 26 befindet sich in der Nähe des in Einführrichtung vorderen Endes der Testträger 2. Der Rastvorsprung 25 hat einen nach unten hin abnehmenden Querschnitt. Sein unteres Ende dringt in eine Vertiefung 19 der Testträgerauflage 20 ein. Die Aufhängung des Rastelementes 24 und der Rastvorsprung 25 sind so ausgebildet und angeordnet, daß sie den Testträger 2 gegen einen Anschlag 27 leicht andrücken und dadurch exakt positionieren.

In der Meßposition wird der Testträger 2 von den Federn 22,23 gegen die Testträgerauflage 20 gedrückt. Die Andruckfedern 22,23 sind so ausgebildet, daß sie beim Einschieben des Testträgers 2 elastisch nach oben nachgeben, ohne daß der Klappdeckel 30 geöffnet werden muß.

Die Positionierung des Testträgers 2 in der Meßposition gemäß Fig. 4 ist sehr wichtig für die Genauigkeit der Auswertung. Um sie auf einfache Weise überprüfen zu können, weist der Testträger 2 die Markierung 13 auf, welche in der Meßposition gegenüber dem Codeleser 42 angeordnet ist.

Die obere Abdeckung der Testträgeraufnahme 7 wird von einem Klappdeckel 30 gebildet, der um Scharniere 31 (Fig. 1) schwenkbar ist. Beim Einführen des Testträgers 2 ist der Klappdeckel 30 geschlossen, wobei die seitliche Führung 21 für den Testträger 2 durch die Wände einer in dem Klappdeckel 30 ausgebildeten Nut 32 gebildet wird. Der aufklappbare Klappdeckel 30 wird vor allem aus den folgenden Gründen benötigt.

Zum einen wird er geöffnet, um einen zu analysierenden Blutstropfen auf das Testfeld 5 aufzugeben. Das Auswertegerät 4 ist nämlich speziell für sogenannte "nicht abzuwischende" Testträger ausgebildet, bei denen die Aufgabe der Blutprobe auf den in der Meßposition befindlichen Testträger erfolgt. Dies stellt eine wesentliche Vereinfachung der Handhabung gegenüber vorbekannten Testträger-Analysesystemen dar, bei denen der Blutstropfen auf einen außerhalb des Gerätes befindlichen Testträger aufgetropft, anschließend eine bestimmte Zeit abgewartet, danach der Blutüberschuß abgewischt oder abgewaschen und schließlich der Testträger in das Gerät eingelegt und ausgewertet werden mußte. Die Erfindung eignet sich in besonderem Maße für Testträger-Analysesysteme die mit nicht abzuwischenden Testträger arbeiten.

Zum zweiten kann der nach der Blutprobenaufgabe verschmutzte Testträger 2 bei geöffnetem Klappdeckel 30 nach oben entnommen werden, ohne die Bauteile der Testträgeraufnahme 7 zu verschmutzen. Um ein versehentliches Herausziehen des Testträgers 2 ohne Öffnen des Klappdeckels 30 zu verhindern, ist das Rastelement 24 in Richtung auf die Einführöffnung 6 (gegen eine Federspannung) beweglich gelagert und darüber ein an dem Klappdeckel 30 befestigtes Sperrelement 33 angeordnet, durch das die Öffnungsbewegung des Rastelementes (nach oben) gesperrt wird, wenn man an dem Testträger 2 in Entnahmerichtung zieht.

Zum dritten läßt sich bei geöffnetem Klappdeckel 30 die Testträgerauflage 20, welche durch eine Rastnase 34 in einer entsprechenden Ausnehmung 35 des Bodenteils 36 herausnehmbar gehalten wird, entnehmen und außerhalb des Gerätes reinigen.

Der Testträger 2 ist in der Meßposition so positioniert, daß die Meßfläche der Auswerteschicht 11 sich exakt gegenüber einem in der Testträgerauflage 20 vorgesehenen Meßfenster 37 befindet, durch das eine remissionsphotometrische Meßvorrichtung 38 die Farbänderung der Auswerteschicht 11 messen kann. Die Meßvorrichtung 38 ist konventionell ausgebildet und kann beispielsweise aus einer Leuchtdiode 39 als Lichtsender und einem Fototransistor 40 als Detektor bestehen. Vorzugsweise erfolgt vor der Probenaufgabe eine Leerwertmessung an der trockenen Nachweisschicht 11.

An dem Einschubweg 28 ist zwischen den seitlichen Führungen 21a, 21b und in Einschubrichtung vor der Meßvorrichtung 38 eine Codelesevorrichtung 42 angeordnet, die zwei seitlich gegeneinander versetzt angeordnete Leseeinheiten 43 und 44 umfaßt. Es handelt sich dabei um in der Zeichnung lediglich schematisch angedeutete miniaturisierte Barcodeleser üblicher Bauart, die auf dem Reflexionsprinzip beruhen und jeweils einen Lichtsender und einen Lichtempfänger enthalten.

Eine Besonderheit besteht darin, daß die Codelesevorrichtung 42 in eine Ausnehmung 20b der Testträgerauflage 20a derartig eindringt, daß ihre Abschlußfläche 45 näherungsweise mit dem sie umgebenden Teil der Oberfläche 20a der Testträgerauflage 20 fluchtet, bzw. diese geringfügig überragt. Dadurch wird die Reinigung erleichtert. Die blockförmige Codelesevorrichtung 42 wird von einem Halteteil 42a in dem Gehäuse des Auswertegerätes 4 gehalten.

Zwischen der Testträgeraufnahme 20 und dem Bodenteil 36 verläuft eine Leiterplatte, die die Auswerteelektronik trägt. Die elektronischen Bauteile können beispielsweise auf der Unterseite der Platine 47 angeordnet sein, wobei das Bodenteil 36 eine entsprechende nicht dargestellte Vertiefung aufweist.

Die insgesamt mit 50 bezeichnete Auswerteelektronik ist in Abbildung 5 lediglich in abstrahierter Form als Block dargestellt. Sie umfaßt eine Meßelektronik 51, an welche die Meßvorrichtung 38 angeschlossen ist und die aus dem Ausgangssignal des Detektors 40 ein Meßsignal erzeugt, eine Codeleseelektronik 52 zur Aufbereitung der Signale der Leseeinheiten 43,44 und eine digitalelektronische Einheit 53, welche einen Mikroprozessor enthält. Die digitalelektronische Einheit 53 weist insbesondere einen Speicher 54 zur Speicherung von Informationen, die von den Leseeinheiten 43,44 gelesen wurden, und eine Vergleichseinheit 55 auf.

Die Codelesevorrichtung 42 dient dazu, sowohl den auf den Codeträgern 3 befindlichen Auswertecode 15, als auch den auf den Testträgern 2 befindlichen Identifikationscode 12 zu lesen. Bevorzugt wird ein Codeträger 3 bis zu dem Anschlag 27 eingeschoben, wobei er von dem Rastelement 24 nicht festgehalten wird, weil er keine Ausnehmung aufweist. Der Auswertecode 15 wird beim Herausziehen gelesen. Die Lesung des Identifikationscodes 12 erfolgt dagegen jeweils beim Einschieben eines Testträgers 2. Der Codeträger 3 muß jeweils eingeschoben und gelesen werden, bevor der erste Testträger 2 der jeweiligen Charge ausgewertet wird. Die in dem Auswertecode 15 codierte Auswerteinformation enthält die chargenspezifische Auswertekurve der Testträgercharge und wird in dem Speicher 54 abgespeichert.

Wenn danach ein Testträger 2 eingeschoben wird, wird sein Identifikationscode 12 gelesen und mit Hilfe der Vergleichsschaltung 55 mit einer Chargenidentifikation verglichen, die ebenfalls in der im Speicher 54 abgespeicherten Auswerteinformation enthalten ist. Wenn beide Chargenidentifikationen übereinstimmen, wird das Meßsignal mit Hilfe der abgespeicherten Auswertekurve in das Analyseergebnis umgewandelt. Stimmt die Charge des eingeschobenen Testträgers dagegen nicht mit der Charge der abgespeicherten Auswertekurve überein, erfolgt keine Auswertung, sondern in dem Display 9 wird ein Hinweis auf den Fehler angezeigt.

Selbstverständlich ist es auch möglich, mehrere unterschiedliche Auswerteinformationen nacheinander einzulesen, wenn der Speicher 54 entsprechend ausgebildet ist. Dies ist besonders zweckmäßig, wenn das Auswertegerät 4 zur Analyse unterschiedlicher Parameter eingesetzt werden soll, die in gemischter Reihenfolge bestimmt werden. In diesem Fall werden die entsprechenden unterschiedlichen Codeträgertypen zweckmäßigerweise nacheinander eingeschoben und die in den Auswertecodes 15 codierten Auswerteinformationen werden abgespeichert. Jedes Mal, wenn ein Testträger 2 eingeschoben wird, werden dessen Typ und Charge anhand des Identifikationscodes 12 erkannt und es erfolgt eine Zuordnung zu der abgespeicherten Auswertekurve des entsprechenden Parameters, wobei auch hier wiederum die Kontrolle der korrekten Chargeninformation in der zuvor beschriebenen Weise möglich ist.

Die Erfindung ermöglicht somit eine einfache Handhabung von Einparameter- oder Mehrparameter-Testträger-Analysesystemen, wobei eine praktisch vollständige Sicherheit gegen Auswertefehler erreicht wird. Dennoch wird der gerätetechnische Aufwand praktisch nicht erhöht, weil eine einzige Codelesevorrichtung sowohl zum Lesen des Identifikationscodes auf den Testträgern als auch zum Lesen des Auswertecodes auf den Codeträgern eingesetzt werden kann.

Die Leseeinheiten 43,44 der Codelesevorrichtung 42 sind seitlich (also in Richtung der Codestreifen) so gegeneinander versetzt, daß sie die Taktspur 16 und die Datenspur 17 des Auswertecodes 15 getrennt erfassen. Ein zweispuriger Code, wie er in Fig. 3 dargestellt ist, erlaubt eine hohe Informationsdichte auf engem Raum und läßt sich zugleich unabhängig von der Einschubgeschwindigkeit lesen. Dies ist wichtig, weil vorzugsweise die Codeträger 3 ebenso wie die Testträger 2 manuell ohne elektromotorische Hilfe in das Auswertegerät 4 eingeschoben werden sollen und weil auf dem Codeträger 3, der der Größe der Testträger 2 entspricht, nur sehr wenig Platz für die Anbringung des Auswertecodes 15 zur Verfügung steht. Die Testträger und die Codeträger haben bevorzugt eine Länge von weniger als 7 cm, besonders bevorzugt höchstens 5 cm und eine Breite von weniger als 6 mm, bevorzugt weniger als 5 mm. Die Breite sollte weniger als 20% der Länge betragen.

Die für die Codierung nutzbare Länge des Codeträgers 3 ist kleiner als der Abstand zwischen dem Rastvorsprung 25 und der Codelesevorrichtung 42. Bei einer praktisch erprobten Ausführungsform der Erfindung beträgt sie nur etwa 2 cm. Im Vergleich zu einem vorbekannten Gerät, bei dem ein verhältnismäßig langer Filmstreifen als Codeträger verwendet wurde, wird hier auf einem Viertel der Codierungsstrecke etwa 60 % der Information untergebracht. Bei einem zweispurigen Code der in Fig. 3 dargestellten Art entspricht jeder Strich und jeder Strichzwischenraum einer Informationseinheit (Bit), deren Inhalt dadurch festgelegt ist, daß an der entsprechenden Stelle der Datenspur ein Codestrich oder kein Codestrich ist. Es gibt also praktisch keine für die Übertragung von Informationen nicht genutzten Zwischenräume.

Der Auswertecode 15 auf den Codeträgern 3 läßt sich verhältnismäßig kostengünstig und in guter Präzision durch Siebdruck aufbringen.

Auch bei den Testträgern 2 steht grundsätzlich die gleiche Länge wie bei den Codeträgern für die Anbringung eines Codes zur Verfügung. Diese Länge wird jedoch zusätzlich verkürzt, wenn - wie bei dem dargestellten Testträger - der Farbumschlag auf der Auswerteschicht 11 auf der gleichen Seite (im vorliegenden Fall der Unterseite) ausgewertet wird, auf der sich auch der Code befindet. Darüber hinaus sind bei der Anbringung von Codes auf Testträgern die in der EP-A-73 056 (entsprechend US-A-4 592 893) näher erläuterten schwierigen Bedingungen zu beachten. Streifenförmige Testträger werden üblicherweise in Form eines langen Produktionsbandes hergestellt, dessen Breite der Länge der Testträger entspricht und auf das ein oder mehrere Testfelder in Form eines schmalen Bandes kontinuierlich aufgebracht werden. Erst am Ende des Produktionsprozesses wird das Produktionsband quer zu seiner Längsrichtung in eine Vielzahl von Teststreifen zerteilt. Für die Anbringung des Strichcodes bedeutet dies, daß er auf die gesamte Länge des Ausgangsbandes (Länge zum Beispiel 200 m) durchlaufend aufgedruckt werden muß. Dabei muß die Genauigkeit des Druckes so gut sein, daß sie eine zuverlässige Lesung des Codes garantiert. Verfahren, mit denen diese Bedingungen erfüllt werden können, sind in den genannten Publikationen beschrieben, sie sind jedoch sehr aufwendig.

Im Rahmen der vorliegenden Erfindung wird als Identifikationscode 12 ein Code verwendet, dessen Codestriche über die gesamte Breite der Testträger 2 verlaufen, so daß das Bedrucken verhältnismäßig einfach und kostengünstig in das übliche Testträgerherstellungsverfahren integriert werden kann.

Obwohl der Identifikationscode 12 ein einfacher einspuriger Code ist, ist er selbsttaktend in dem Sinne, daß er unabhängig von der Einschubgeschwindigkeit zuverlässig gelesen werden kann. Dies wird anhand von Fig. 6 erläutert.

Der Deutlichkeit halber zeigt Fig. 6 die Leseeinheiten 43 und 44 neben dem entlang des Einschubwegs 28 eingeschobenen Identifikationscode 12, während sie in Wahrheit selbstverständlich unterhalb des Codes angeordnet sind. Die Leseeinheiten 43,44 sind in Richtung des Einschubweges 28 (also in Leserichtung des Codes) gegeneinander versetzt. Der Identifikationscode 12 besitzt Codestriche unterschiedlicher Breite. Der Abstand der Leseeinheiten 43,44 und die Breite und Sequenz der Codestriche sind so aufeinander abgestimmt, daß beide Leseeinheiten entweder den gleichen Zustand ("schwarz" bzw. "weiß") sehen oder einen unterschiedlichen Wert. Dies entspricht den Bit-Werten 0 und 1 und ist unabhängig von Schwankungen der Einschubgeschwindigkeit. In der in Fig. 6 dargestellten Position "sieht" beispielsweise die Leseeinheit 44 einen Balken, 43 einen Balkenzwischenraum. Nach kurzer Verschiebung in Richtung 28 "sieht" 44 einen Zwischenraum und 43 einen Balken. Bei weiterer Verschiebung steht der mittlere breite Balken beiden Leseeinheiten gegenüber, so daß beide "schwarz" sehen. Selbstverständlich ist auch ein Zustand, bei dem beide Leseeinheiten "weiß" sehen möglich, jedoch nicht dargestellt. Insgesamt können bei einem solchen Codierungsverfahren also gewünschtenfalls vier verschiedene Zustände unabhängig von Schwankungen der Einschubgeschwindigkeit zuverlässig dargestellt werden.

Wenn die Leseeinheiten 43,44 der Codelesevorrichtung 42 wie dargestellt in Einschubrichtung gegeneinander versetzt sind, muß diese Versetzung selbstverständlich bei der Gestaltung des zweispurigen Codes gemäß Fig. 3 berücksichtigt werden. Dies ist jedoch problemlos möglich.

## Patentansprüche

1. Testträger-Analysesystem zur Analyse eines Bestandteils einer flüssigen Probe, insbesondere einer Körperflüssigkeit, umfassend
Testträger (2), welche in einer oder mehreren Testschichten Reagenzien enthalten, wobei die Reaktion der Reagenzien mit dem Bestandteil zu einer physikalisch nachweisbaren Veränderung einer Nachweisschicht (11) führt, die Veränderung in einem bestimmten, einer Auswertekurve folgenden Zusammenhang zu der Konzentration des Bestandteils steht, und die Auswertekurve von der Herstellungscharge des Testträgers (2) abhängig ist,
Codeträger (3), welche einen Auswertecode (15) aufweisen, der in maschinenlesbarer Form eine Auswerteinformation enthält, die die chargenspezifische Auswertekurve einschließt, und
ein Auswertegerät (4), das eine Testträgeraufnahme (7) zur Positionierung eines Testträgers (2) in einer Meßposition, eine Meßvorrichtung (38) zur Messung der physikalisch nachweisbaren Veränderung und Erzeugung eines Meßsignals, eine Codelesevorrichtung (42) zum Lesen des auf dem Codeträger (3) enthaltenen Auswertecodes (4), einen Speicher (54) zur Speicherung der in dem Auswertecode (15) codierten Auswerteinformation, und eine Auswerteelektronik (50) zur Umwandlung des Meßsignals unter Berücksichtigung der gespeicherten Informationen über die chargenspezifische Auswertekurve in ein Analyseergebnis aufweist,
wobei
der Auswertecode (15) auf den Codeträgern (3) ein zweispuriger Barcode mit getrennter Taktspur (16) und Datenspur (17) ist, und die darin codierte Auswerteinformation eine Chargenidentifikation enthält,
die Testträger (2) jeweils einen chargenspezifischen Identifikationscode (12) aufweisen, der als Barcode mit über die gesamte Breite der Testträger (2) verlaufenden Codestrichen ausgebildet ist,
die Codelesevorrichtung (42) an der Testträgeraufnahme (7) so angeordnet ist, daß sowohl ein Testträger (2) als auch ein Codeträger (3) wahlweise eingeschoben und deren Codes (12,15) gelesen werden können,
die Codelesevorrichtung (42) zwei sowohl seitlich als auch in Richtung der Einschiebbewegung gegeneinander versetzte Leseeinheiten (43,44) aufweist, und
das Auswertegerät (4) eine Vergleichseinheit (55) zum Vergleich des Identifikationscodes der Testträger (2) und der Chargenidentifikation der Auswertekurve und damit zur Überprüfung der Zuordnung der Charge des jeweils auszuwertenden Testträgers und der abgespeicherten Auswerteinformation aufweist.

2. Analysesystem nach Anspruch 1, bei dem die Testträger (2) und die Codeträger (3) streifenförmig ausgebildet sind.

3. Analysesystem nach einem der vorhergehenden Ansprüche, bei dem die Abmessungen der Testträger (2) und der Codeträger (3) so aufeinander abgestimmt sind, daß sie gemeinsam verpackt werden können.

4. Analysesystem nach einem der vorhergehenden Ansprüche, bei dem die Testträger (2) eine Markierung (13) an einer Stelle aufweisen, die in der Meßposition gegenüber der Codelesevorrichtung (42) angeordnet ist, um die korrekte Positionierung des Testträgers zu prüfen.

5. Analysesystem nach einem der vorhergehenden Ansprüche, bei dem die Testträgeraufnahme (7) eine herausnehmbare Testträgerauflage (20) aufweist, welche mit einer Ausnehmung (20b) versehen ist, in die die Codelesevorrichtung (42) im eingesetzten Zustand der Testträgerauflage derartig eindringt, daß ihre obere Abschlußfläche (45) und die Oberfläche (20a) der Testträgerauflage (20) näherungsweise fluchten.

6. Analysesystem nach einem der vorhergehenden Ansprüche, bei dem verschiedene Testträgertypen mit verschiedenen Reagenzien zur Bestimmung verschiedener Probenbestandteile und jeweils den Testträgertypen zugeordnete Codeträgertypen vorgesehen sind und der Identifikationscode (12) auf den Testträgern (2) und der Auswertecode (15) auf den Codeträgern (3) jeweils Informationen über den Testträgertyp enthalten.

7. Analysesystem nach einem der vorhergehenden Ansprüche, bei dem die Testträger (2) und die Codeträger (3) streifenförmig ausgebildet sind und eine Länge von weniger als 7 cm, bevorzugt weniger als 5 cm haben.

8. Analysesystem nach einem der vorhergehenden Ansprüche, bei dem die Testträger (2) und die Codeträger (3) eine Breite von weniger als 6 mm, bevorzugt weniger als 5 mm haben.

9. Analysesystem nach einem der vorhergehenden Ansprüche, bei dem die Breite der Testträger (2) und der Codeträger (3) weniger als 20% ihrer Länge beträgt.

## Claims

1. Test carrier analysis system for analyzing a constituent of a fluid sample, in particular of a body fluid, comprising
test carriers (2) which contain in one or more test layers reagents, in which the reaction of the reagents with the constituent leads to a physically detectable change in a detection layer (11), the change follows a particular relationship, in accordance with an evaluation curve, to the concentration of the constituent, and the evaluation curve is dependent on the manufacturing batch of the test carrier (2),
code carriers (3) having an evaluation code (15) which contains in machine-readable form an item of evaluation information which includes the batch-specific evaluation curve, and
an evaluation device (4) which comprises a test carrier receiver (7) for positioning a test carrier (2) in a measuring position, a measuring device (38) for measuring the physically detectable change and generating a measurement signal, a code reading device (42) for reading the evaluation code (4) contained on the code carrier (3), a storage means (54) for storing the evaluation information coded in the evaluation code (15), and evaluation electronics (50) for converting the measurement signal in accordance with the stored items of information by means of the batch-specific evaluation curve into an analysis result,
in which
the evaluation code (15) on the code carriers (3) is a two-track bar code with separate clock track (16) and data track (17), and the evaluation information coded therein contains a batch identification,
the test carriers (2) have a batch-specific identification code (12) which is formed as a bar code with code bars running across the whole width of the test carriers (2),
the code reading device (42) is arranged on the test carrier receiver (7) so that both a test carrier (2) and a code carrier (3) can optionally be inserted and their codes (12, 15) can be read,
the code reading device (42) comprises two reading units (43, 44) offset relative to one another both laterally and in the direction of the insertion movement, and
the evaluation device (4) comprises a comparison unit (55) for comparing the identification code of the test carriers (2) with the batch identification of the evaluation curve and hence for checking the correlation of the batch of the respective test carrier to be evaluated with the stored evaluation information.

2. Analysis system according to claim 1, in which the test carriers (2) and the code carriers (3) are designed in strip form.

3. Analysis system according to one of the preceding claims, in which the dimensions of the test carriers (2) and the code carriers (3) are coordinated with one another in such a way that they can be packed together.

4. Analysis system according to any one of the preceding claims, in which the test carriers (2) comprise a mark (13) at a point which in the measuring position is arranged opposite the code reading device (42), in order to check the correct positioning of the test carrier.

5. Analysis system according to any one of the preceding claims, in which the test carrier receiver (7) comprises a removable test carrier support (20) which is provided with a recess (20b) into which the code reading device (42) penetrates when the test carrier support is in the inserted position, in such a way that its upper surface (45) and the surface (20a) of the test carrier support (20) are approximately aligned.

6. Analysis system according to any one of the preceding claims, in which different types of test carrier containing different reagents for determining different sample constituents and respective code carrier types correlated with the test carrier types are provided and the identification code (12) on the test carriers (2) and the evaluation code (15) on the code carriers (3) each contain information on the type of test carrier.

7. Analysis system according to any one of the preceding claims, in which the test carriers (2) and the code carriers (3) are strip-shaped and have a length of less than 7 cm, preferably less than 5 cm.

8. Analysis system according to any one of the preceding claims, in which the test carriers (2) and the code carriers (3) have a width of less than 6 mm, preferably less than 5 mm.

9. Analysis system according to any one of the preceding claims, in which the width of the test carriers (2) and code carriers (3) amounts to less than 20% of their length.

## Revendications

1. Analyseur pour bandes de test pour l'analyse d'un composant d'un prélèvement liquide, en particulier d'un corps liquide, comprenant des bandes de test (2) qui renferment des réactifs en une ou plusieurs couches de test, la réaction des réactifs conduisant avec le composant à une modification, décelable physiquement, d'une couche de décèlement (11), la modification se trouvant dans une relation déterminée et suivant une courbe d'évaluation, avec la concentration du composant et la courbe d'évaluation étant dépendante du lot de fabrication de la bande de test (2), des bandes-code (3) qui présentent un code d'évaluation (15) qui contient une information d'évaluation sous forme exploitable par une machine et incluant la courbe d'évaluation spécifique au lot, et un appareil d'analyse (4) qui présente un logement de bandes de test (7) destiné au positionnement d'une bande de test (2) dans une position de mesure, un dispositif de mesure (38) pour mesurer la modification et la production décelables physiquement d'un signal de mesure, un dispositif de lecture de code (42) pour lire le code d'évaluation (4) figurant sur la bande-code (3), une mémoire (54) pour mémoriser l'information d'évaluation codée dans le code d'évaluation (15), et une électronique de traitement (50) destinée à convertir le signal de mesure en un résultat d'analyse, en prenant en compte les informations mémorisées relatives à la courbe d'évaluation spécifique au lot, le code d'évaluation (15) figurant sur les bandes-code (3) étant un code à barres à deux pistes avec une piste de temporisation (16) et une piste de données (17) séparées, et l'information d'évaluation qui y est codée renfermant une identification de lot, chaque bande de test (2) présentant un code d'identification (12) spécifique du lot, code qui est conçu en tant que code à barres avec des barres couvrant toute la largeur des bandes de test (2), le dispositif de lecture de code (42) étant disposé sur le logement des bandes de test (7) de façon à ce qu'aussi bien une bande de test (2) qu'une bande-code (3) puissent être introduites à volonté et que leurs codes (12, 15) puissent être lus, le dispositif de lecture de code (42) présentant deux unités de lecture (43, 44) décalées l'une par rapport à l'autre, aussi bien latéralement que dans le sens du mouvement d'introduction, et l'appareil d'analyse (4) présentant une unité de référence (55) pour comparer le code d'identification des bandes de test (2) et l'identification du lot de la courbe d'évaluation et donc pour contrôler l'affectation du lot de chaque bande de test à analyser et de l'information d'évaluation mémorisée.

2. Analyseur selon la revendication 1, pour lequel les bandes de test (2) et les bandes-code (3) sont conçues en forme de barres.

3. Analyseur selon l'une des revendications précédentes, pour lequel les dimensions des bandes de test (2) et des bandes-code (3) correspondent les unes aux autres de telle sorte qu'elles peuvent être emballées ensemble.

4. Analyseur selon l'une des revendications précédentes, pour lequel les bandes de test (2) présentent une marque (13) à un emplacement qui se trouve, dans la position de mesure, en face du dispositif de lecture de code (42) pour contrôler le positionnement correct de la bande de test.

5. Analyseur selon l'une des revendications précédentes, pour lequel le logement des bandes de test (7) présente un support détachable (20) qui est muni d'un creux (20b) dans lequel, lorsque le support des bandes de test est inséré, le dispositif de lecture de code (42) pénètre de telle sorte que sa surface supérieure de fermeture (45) et la surface (20a) du support des bandes de test (20) s'alignent approximativement.

6. Analyseur selon l'une des revendications précédentes, pour lequel sont prévus différents types de bandes de test avec différents réactifs pour déterminer différents composants de prélèvements ainsi que des types de bandes-code affectés à chaque fois à des types de bandes de test, et le code d'identification (12) figurant sur les bandes de test (2) ainsi que le code d'évaluation (15) figurant sur les bandes-code (3) contiennent chacun des informations relatives au type de bande de test.

7. Analyseur selon l'une des revendications précédentes, pour lequel les bandes de test (2) et les bandes-code (3) sont conçues en forme de barres et ont une longueur inférieure à 7 cm et, de préférence, inférieure à 5 cm.

8. Analyseur selon l'une des revendications précédentes, pour lequel les bandes de test (2) et les bandes-code (3) ont une largeur inférieure à 6 mm et, de préférence, inférieure à 5 cm.

9. Analyseur selon l'une des revendications précédentes, pour lequel la largeur des bandes de test (2) et des bandes-code (3) est inférieure à 20 % de leur longueur.
